# EUROPEAN PATENT SPECIFICATION

(11) **EP 2 865 390 B1**
(45) Date of publication and mention of the grant of the patent: **21.08.2019**
(21) Application number: 13806404.3
(22) Date of filing: 19.06.2013
(51) Int. Cl.: A61K 45/00, A61K 35/74, A61K 36/25, A61K 38/00, A61K 39/12, A61K 39/39, A61P 35/00, A61P 37/04, C07K 14/01, C12N 1/20, C12N 1/21, C12N 15/09

(54) **MUCOSAL IMMUNITY-STIMULATING AGENT, AND ORAL PHARMACEUTICAL COMPOSITION FOR TREATING HPV INFECTION**
MUKOSALES IMMUNITÄTSSTIMULIERENDES MITTEL UND ORALE PHARMAZEUTISCHE ZUSAMMENSETZUNG ZUR BEHANDLUNG VON HPV-INFEKTIONEN
AGENT DE STIMULATION DE L'IMMUNITÉ MUQUEUSE ET COMPOSITION PHARMACEUTIQUE ORALE PERMETTANT DE TRAITER UNE INFECTION PAR LE VPH

(30) Priority: 20.06.2012 JP 2012138943
(43) Date of publication of application: 29.04.2015
(73) Proprietor: The University of Tokyo, Bunkyo-ku, Tokyo 113-8654 (JP)
(72) Inventor: KAWANA, Kei, Tokyo 113-8654 (JP); TAGUCHI, Ayumi, Tokyo 113-8654 (JP)
(74) Representative: Graf von Stosch, Andreas
(86) International application number: PCT/JP2013/066893
(87) International publication number: WO 2013/191225

(56) References cited:
- WO-A2-2005/000348
- JP-A- 2001 523 729
- JP-A- 2005 500 336
- MATSUMOTO T1 ET AL: "Hochuekkito, a Kampo (traditional Japanese herbal) Medicine, Enhances Mucosal IgA Antibody Response in Mice Immunized with Antigen-entrapped Biodegradable Microparticles", EVIDENCE-BASED COMPLEMENTARY AND ALTERNATIVE MEDICINE : ECAM, HINDAWI PUBLISHING CORPORATION, UNITED STATES, vol. 7, no. 1, 29 October 2007 (2007-10-29), pages 69-77, XP008175992, ISSN: 1741-427X, DOI: 10.1093/ECAM/NEM166
- KATSUYUKI ADACHI ET AL: "Oral immunization with a Lactobacillus casei vaccine expressing human papillomavirus (HPV) type 16 E7 is an effective strategy to induce mucosal cytotoxic lymphocytes against HPV16 E7", VACCINE, vol. 28, no. 16, 1 April 2010 (2010-04-01) , pages 2810-2817, XP055182241, ISSN: 0264-410X, DOI: 10.1016/j.vaccine.2010.02.005
- VANUSA P DA HORA ET AL: "Non-toxic derivatives of LT as potent adjuvants", VACCINE, ELSEVIER LTD, GB, vol. 29, no. 8, 30 November 2010 (2010-11-30), pages 1538-1544, XP028139969, ISSN: 0264-410X, DOI: 10.1016/J.VACCINE.2010.11.091 [retrieved on 2010-12-06]
- FREYTAG L C ET AL: "Mucosal adjuvants", VACCINE, ELSEVIER LTD, GB, vol. 23, no. 15, 7 March 2005 (2005-03-07) , pages 1804-1813, XP027652249, ISSN: 0264-410X [retrieved on 2005-03-07]
- HARANDI ALI M ET AL: "Mucosal Adjuvants", CURRENT HIV RESEARCH, BENTHAM SCIENCE PUBLISHERS, HILVERSUM, NL, vol. 8, no. 4, 1 June 2010 (2010-06-01), pages 330-335, XP009185085, ISSN: 1570-162X
- CHINO A ET AL: "Juzentaihoto, a Kampo medicine, enhances IL-12 production by modulating Toll-like receptor 4 signaling pathways in murine peritoneal exudate macrophages", INTERNATIONAL IMMUNOPHARMACOLOGY, ELSEVIER, AMSTERDAM, NL, vol. 5, no. 5, 1 May 2005 (2005-05-01), pages 871-882, XP027686239, ISSN: 1567-5769 [retrieved on 2005-05-01]
- MITA Y ET AL: "Surface expression of toll-like receptor 4 on THP-1 cells is modulated by Bu-Zhong-Yi-Qi-Tang and Shi-Quan-Da-Bu-Tang.", METHODS AND FINDINGS IN EXPERIMENTAL AND CLINICAL PHARMACOLOGY MAR 2002, vol. 24, no. 2, March 2002 (2002-03), pages 67-70, XP009188623, ISSN: 0379-0355
- JERRY WELLS: "Mucosal Vaccination and Therapy with Genetically Modified Lactic Acid Bacteria", ANNUAL REVIEW OF FOOD SCIENCE AND TECHNOLOGY, vol. 2, no. 1, 10 April 2011 (2011-04-10), pages 423-445, XP055250437, US ISSN: 1941-1413, DOI: 10.1146/annurev-food-022510-133640
- MATSUMOTO T ET AL.: 'Hochuekkito, a Kampo (traditional Japanese herbal) Medicine, Enhances Mucosal IgA Antibody Response in Mice Immunized with Antigen-entrapped Biodegradable Microparticles.' EVID BASED COMPLEMENT ALTERNAT MED. vol. 7, no. 1, 29 October 2007, pages 69 - 77, XP008175992
- KOICHIRO SAITO: 'Koto Nyutoshusho' JOHNS vol. 27, no. 9, 01 September 2011, pages 1460 - 1462, XP008175909
- CYONG J ET AL.: 'Immunomodulatory action of Kampo tonics and its potential mode of action in aged mice, Journal of traditional medicines' vol. 15, no. 5, 27 February 1999, pages 280 - 281, XP008175991
- NORITO TAKEMOTO ET AL.: 'Effect of TJ-48 on murine cellular immunity' JAPANESE JOURNAL OF INFLAMMATION vol. 9, no. 1, 1989, pages 49 - 52, XP003034247
- MATSUMOTO T ET AL.: 'Orally administered Kampo (Japanese herbal) medicine, ''Juzen-Taiho- To'' modulates cytokine secretion in gut associated lymphoreticular tissues in mice.' PHYTOMEDICINE. vol. 6, no. 6, January 2000, pages 425 - 430, XP055182238
- ADACHI K ET AL.: 'Oral immunization with a Lactobacillus casei vaccine expressing human papillomavirus (HPV) type 16 E7 is an effective strategy to induce mucosal cytotoxic lymphocytes against HPV16 E7.' VACCINE. vol. 28, no. 16, 17 February 2010, pages 2810 - 2817, XP055182241
- TAGUCHI A ET AL.: 'Adjuvant effect of Japanese herbal medicines on the mucosal type 1 immune responses to human papillomavirus (HPV) E7 in mice immunized orally with Lactobacillus-based therapeutic HPV vaccine in a synergistic manner.' VACCINE. vol. 30, no. 36, 21 June 2012, pages 5368 - 5372, XP028428427

## Description

### Technical Field

The present invention relates to an oral pharmaceutical composition and its use in a method for treating HPV infection. More particularly, the present invention relates to an oral pharmaceutical composition containing a HPV E7 polypeptide, a mucosal adjuvant and a Kampo preparation having a revitalizing activity, and an oral pharmaceutical composition for use in a method of treating HPV infection containing a HPV E7 polypeptide, a mucosal adjuvant and a Kampo preparation having a revitalizing activity.

### Background Art

A human papillomavirus (HPV) is a DNA virus. To date, there have been reported 100 or more different types of the HPVs. The HPV infects the skin (e.g., HPV-1 and HPV-2) and the mucosal surface (e.g., HPV-6 and HPV-11) to thereby form a benign tumor (wart) existing for from a few months to a few years.

Additionally, some of the HPVs (e.g., HPV-16, HPV-18, HPV-31, and HPV-33) infect the mucosa for a long period of time to thereby cause a malignant tumor.

Almost 100% of cervical cancers, which have the second highest morbidity next to breast cancers among cancers specific to women, is developed by infection of the HPV in reproductive organs for a long period of time. It is reported that 500,000 people in the world and 15,000 people in Japan newly develop the cervical cancer every year.

The number of deaths per year from the cervical cancer reaches 270,000 in the world and 3,500 in Japan.

Theoretically speaking, if the HPV infection is completely prevented, the cervical cancer can be eradicated.

The HPV infection has been revealed to be associated with vulvar cancer, anal cancer, oral cancer, vaginal cancer, penile cancer, pharyngeal cancer, and condyloma acuminatum (venereal wart), in addition to the cervical cancer. Therefore, a prophylactic vaccine against the HPV infection is thought to be effective for prevention of above-described cancers and sexually transmitted diseases.

Among the HPVs, HPV16 and HPV 18 accounts for about 65% of cause for the cervical cancer in Japanese women, especially about 90% in their twenties.

The HPV16 and the HPV18 have also been known to account for a little less than 50% of cause for high-grade lesions of vulvar intraepithelial neoplasia which precede the development of the vulvar cancer, and to be associated with 85% of the development of vaginal intraepithelial neoplasia which may proceed to the vaginal cancer.

Nowadays, as a prophylactic vaccine against the HPV infection, especially against the cervical cancer, there are used a bivalent vaccine specific for the HPV16 and the HPV18 (trademark name: CERVARIX) (see PTL 1) and a tetravalent vaccine specific for, in addition to the HPV16 and the HPV18, HPV6 and HPV11 which account for about 90% of cause for the condyloma acuminatum (trademark: GARDASIL) (see PTL 2). Both of these two vaccines contain virus-like particles of L1 protein from the HPV.

However, unless a prophylactic vaccine against a wider range of the HPVs is developed and it is applied to 100% of adolescent HPV-uninfected women around the world, it cannot be expected that patients with the cervical cancer and precursor lesions thereof will become unexistent. Therefore, at present, huge cost is expended on only cervical cytodiagnosis.

A HPV E7 protein has a binding ability with an Rb protein in vitro, and it binds with the Rb protein to thereby inhibit a binding of a transcription factor E2F with the Rb protein. As a result, the E2F which is unbound to the Rb protein is activated, leading to a cell cycle transition from G1 phase to S phase.

Meanwhile, an E6 protein, which is another HPV protein, binds to a p53 protein to thereby promote its ubiquitination, so that the p53 protein is degraded.

From the above, it is presumed that, by the function of the E7 protein and the E6 protein, HPV-infected cells avoid apoptosis which is regulated by a cell cycle checkpoint mechanism and the p53 protein, to thereby cancerate.

Actually, HPV E7 and E6 mRNAs are constitutively overexpressed in cervical cancer cells.

When the HPV E6 and E7 in the cervical cancer cells are suppressed in expression by, for example, an antisense method, the cervical cancer cells are suppressed in proliferative activity.

In addition, it has been shown that the cervical cancer cells in which the E6 and the E7 are suppressed in expression by, for example, the antisense method have lost their tumorigenicity when transplanted into a mouse.

From the above, it is obvious that the HPV E6 and E7 are functionally highly associated with pathologies and carcinogenesis processes of the cervical cancer and other HPV-associated cancers.

Many of cervical intraepithelial neoplasia (CIN), which is a cervical cancer precursor lesion resulting from the HPV infection are formed in a zone which is called as a uterine junctional zone. In the junctional zone, upon restoration of erosion in the vaginal portion of cervix (i.e., a lower half part of the uterine cervix, a part protruding to the vagina), squamous metaplasia (squamatization of reserve cells in the vagina) occurs. It is believed that the epidermal basal cells formed at that time may be infected with HPV, resulting in the cervical cancer.

HPV-infected epidermal basal cells each have as few as several ten to one hundred copies of HPV DNA and the E6 and the E7 are underexpressed therein.

Typically, at an early stage of the HPV infection in which the E6 and the E7 are underexpressed, many of the HPVs including the HPV which belongs to a high risk group for onset of the cervical cancer are in a latent state in which the HPVs stop to proliferate.

Centers for Disease Control and Prevention have been reported that 0.13% or less of the HPV infections in the uterine cervix proceeds to an invasive cancer.

Meanwhile, in a developmental process of the cervical cancer, the following changes are thought to occur: the HPV continuously spreads to the basal cells, and the E6 and the E7, which was underexpressed, are continuously increased in expression.

The E6 and the E7 are very lowly expressed in CIN1 (mild dysplasia), but highly expressed in CIN2 (moderate dysplasia) and CIN3 (severe dysplasia). Therefore, it is believed to be at the CIN1 stage that the E6 and the E7 are continuously increased in expression as described above.

The HPV infection is believed to be controlled by a host immune response. Actually, it has been reported that an incidence rate of the cervical cancer is high in women with reduced immune response such as HIV-infected patients and patients who had been subjected to organ transplantation.

The host immune response against a virus (e.g., HPV) includes an unspecific mechanism called as innate immunity which works from immediately after infection by the action of interferon and cell groups (e.g., NK cell and macrophage) and, apart from that, a viral antigen-specific immune response called as acquired immunity.

The acquired immunity is an immune mechanism which works from several days after infection, and is classified into humoral immunity and cellular immunity.

An immune response which participates in elimination of the HPV is believed to be mainly composed of a cellular response by the NK cell and a cytotoxic T cell (CTL) which participates in the cellular immunity.

The NK cell is an immune cell working at the early stage of the HPV infection.

Meanwhile, regarding a HPV-specific cellular immunity, it has been shown that a delayed type allergic reaction against the HPV16 E7 protein mutually relates to CIN regression and that an E7-specific helper T cell plays an important role in control of the HPV infection (see NPL 1).

As mentioned above, the HPV E6 protein and the HPV E7 protein, especially the HPV E7 protein, are believed to be an attractive target in a development of a therapeutic vaccine against HPV-associated cancers.

Although there have already been developed some therapeutic vaccines using as an antigen the HPV E7 protein, they have not yet demonstrated to eliminate the precursor lesion (the CIN) and cancer cells in the uterine cervix of a patient. Therefore, there is a strong need to develop a vaccine which has been demonstrated to have a therapeutic effect (see NPLs 2 to 4).

Recently, the present inventors have found that an E7-specific cellular immune response can be elicited in not only the spleen but also the mucosa in a mouse through orally administration of a vaccine (LacE7) which is made by expressing a mutant E7 protein in *Lactobacillus casei,* followed by subjecting to heat treatment to kill and drying to powder (see NPL 5). The mutant E7 protein is the HPV16 E7 protein in which Asp at position 21, Cys at position 24, and Glu at position 26 involving in binding with the Rb protein are all substituted with Gly.

Additionally, the present inventors examined a LacE7 preparation for safety and pathological efficacy through the exploratory clinical study against the precursor lesion of the cervical cancer (CIN).

Elicitation of an E7-specific cellular immunity in the cervical lymphocyte and the peripheral blood was also examined.

Seven patients with a HPV16-positive precursor lesion (CIN3) were assigned to a 1 capsule/day administered group (n=1), a 2 capsules/day administered group (n=3), and a 4 capsules/day administered group (n=3), and administered with the LacE7 preparation for a total of 4 rounds at weeks 1, 2, 4, and 8 with one round being composed of oral administration of 1 capsule (250 mg) once a day for 5 days.

Consequently, the patients of the 1 capsule/day administered group and the 2 capsules/day administered group had to be subjected to conization after the completion of the study. However, in the patients of the 4 capsules/day administered group, the CIN3 was completely disappeared and no exacerbation occurred thereafter.

In the immunological analysis, elicitation of the E7-specific cellular immunity against the HPV16 in the cervical lymphocyte (induction of E7-specific IFN-γ-producing cells and E7-specific granzyme B-producing cells) was observed.

Pathologically, considerable infiltration of lymphocytes into the precursor lesion was observed, which had not been observed in the precursor lesion before administration.

Therefore, the LacE7 which was developed by the present inventors is expected to be the first therapeutic vaccine for patients with the precursor lesion (CIN3).

However, there has not yet been found a satisfactory therapeutic vaccine against HPV having a sufficient clinical effect for not only patients with the precursor lesion but also cervical cancer patients.

That is, there has still been a problem that there is no therapeutic vaccine against HPV having a sufficient clinical effect for cervical cancer patients.

There has not been developed a method for effectively eliciting acquired immunity against the HPV through oral administration of the LacE7 in a smaller amount and for a shorter period or oral administration of a vaccine against HPV other than the LacE7; or a method for eliciting a HPV-specific cellular immunity on, in particular, the mucosa, which is also problematic.

Many of pathogens including viruses (e.g., the HPV or HIV) invade a host through the mucosa to thereby establish infection. Therefore, a mucosal immune system in the local mucosa is believed to play an important role in an infection protective mechanism of the host.

A secretory IgA antibody plays an important role in humoral immunity in the local mucosa.

Production of the IgA antibody is regulated by a B cell, a T cell, a transforming growth factor (TGF)-β secreted from macrophage, and a cytokine (e.g., interleukin (IL)-4 and IL-6). These humoral factors are functionally important in maintenance and modification of the humoral immunity on the mucosa.

In recent years, it has been found that, as immunity on the local mucosa, a cellular immune response in which a cytotoxic T cell (CTL) plays a central role is elicited on the mucosae in the digestive tract and the reproductive organ. For example, interferon (IFN)-y participates in activation of the CTL which is specific for an antigen.

Juzentaihoto and Hochuekkito, which are Kampo preparations, and a mucosal adjuvant have already been known to be a mucosal immunity-stimulating agent stimulating humoral immunity, which is one of acquired immunities, on the mucosa.

Among Kampo preparations (Wakan crude drugs), there is a family of crude drug used for treating infection as described in "Treatise on Cold Damage Disorders" which is an article regarding Kampo medicine.

In modern times, the Kampo preparation has been used for treating various infections and infection-associated diseases (e.g., common cold, influenza, viral hepatitis, and Helicobacter pylori infection), and has been shown its efficacy. It may act directly against a pathogenic virus or a pathogenic bacterium, but often through enhancement of a biological defense mechanism via an immunoregulatory activity.

In the modern medicine, antibiotics and vaccines are generally used for treatment of bacterial infection and prophylaxis of viral infection, respectively. On the other hand, some of the Kampo preparations have been known to have a prophylaxis effect through enhancement of immunocompetence of a host.

For example, in the case of influenza infection, Kakkonto has not a direct proliferation suppression effect against an influenza virus, but exhibits therapeutic effects such as antipyresis, amelioration of pneumonia, and a decrease of mortality.

When mice are transnasally infected with the influenza virus, an IFN level in the serum and, in turn, an IL-1α level are increased to thereby increase an intracerebral cyclooxygenase activity. As a result, a prostaglandin E₂ level is increased, resulting in a development of a fever.

An aspirin inhibits the cyclooxygenase. On the other hand, the Kakkonto suppresses production of the IL-1α induced by the IFN to thereby exert an antipyretic activity.

Juzentaihoto and Hochuekkito are Kampo medicines used for treatment of general malaise and anorexia upon postoperative, convalescent, or postpartum exhaustion, exhaustion from chronic diseases, debilitation from accumulation of fatigue and summer emaciation. The Juzentaihoto is also used for anemia. On the other hand, the Hochuekkito is used for improvement of physical constitutions and amelioration of symptoms such as a slight fever, a cough, a night sweat, and palpitations which symptoms are developed when a cold lasts long or when frail people catches a cold.

Both of the Juzentaihoto and the Hochuekkito have been demonstrated to activate a humoral immune mechanism in which a B cell participates, in the mucosae of the intestinal tract and the trachea (see PTL 3 and NPL 6). Hochuekkito has been shown to enhance the mucosal IgA antibody response in mice immunized with antigen-entrapped biodegradable microparticles (NPL12). That is, the Juzentaihoto and the Hochuekkito have been revealed to be a mucosal immunity-stimulating agent which stimulates humoral immunity on the mucosa.

Other Kampo preparations such as Shosaikoto and Shoseiryuto may also be used for treatment and prophylaxis of infections. These are also believed to activate an immune mechanism of a host.

Those generally referred to as "adjuvant" in immunology are reagents used for enhancement of antigenicity (immunological enhancement) by injecting together with an antigen. The adjuvant is believed to act, by way of example, as follows, but there are various mechanisms of action, many of which are unknown. Firstly, the adjuvant insolubilizes the antigen to thereby keep for a long period, leading to a long-time sustained release of the antigen. Secondly, the adjuvant causes inflammation in a site to which the antigen has been administered, which mobilizes macrophage. As a result, the antigen is likely to be subjected to phagocytosis, which promotes antigen presentation. Thirdly, the adjuvant activates immune cells.

Kampo preparations having a so-called revitalizing activity such as the Kakkonto, the Juzentaihoto, the Hochuekkito, the Shosaikoto, and the Shoseiryuto are also included in the adjuvant in a broad sense from the viewpoint of having a humoral immunity-enhancing effect.

Examples of well known mucosal adjuvants include a cholera toxin (CT) from bacteria, a heat-labile enterotoxin (LT) from enterotoxigenic *E. coli,* a verotoxin (VT) from enterohemorrhagic *E. coli,* a diphteria toxin (DT), and a pertussis toxin (PT). These have strong immunogenicity and are the most effective adjuvants experimentally.

All of the mucosal adjuvants from bacteria have been revealed to exhibit an excellent mucosal and systemic adjuvant effect, but cause severe side effects such as optic nerve paralysis. Therefore, they cannot be used for treatment of human as they are.

Accordingly, a mutant mucosal adjuvant which can avoid the side effects was newly developed recently and is available.

In addition to the wild-type and mutant mucosal adjuvants from bacteria, examples of mucosal adjuvants which cause no side effects and can be clinically used include α-galactosylceramide (aGalCer) which is a synthetic ceramide; a CpG oligonucleotide; pulmonary surfactant protein-C (SP-C, may be referred to as SURFACTEN) and pulmonary surfactant protein-B (SP-B) which are biosurfactants consisting of phospholipids and apoproteins; a wild-type or mutant IFN-α; a double-strand RNA; and an activity-enhanced TNF mutant (see PTLs 4 to 6 and NPLs 7 to 11).

However, in protection against viral (e.g., HPV) and bacterial infection or treatment of infections, there has not yet been found a satisfactory mucosal immunity-stimulating agent intended to be administered in combination with a vaccine against the virus and the bacterium.

That is, there has still been a problem that there is no oral pharmaceutical composition containing a mucosal immunity-stimulating agent which has a sufficient cellular immunity-stimulating effect on the mucosa and can be administered in combination with a viral vaccine such as a HPV vaccine and a bacterial vaccine.

### Citation List

### Patent Literature

PTL 1: International Publication No. WO 2003/078455
PTL 2: International Publication No. WO 2000/045841
PTL 3: Japanese Patent Application Laid-Open (JP-A) No. 2006-070217
PTL 4: International Publication No. WO 2007/018152
PTL 5: International Publication No. WO 2004/028561
PTL 6: JP-A No. 2005-097267

### Non-Patent Literature

NPL 1: R. Hopfl et al., Lancet, 2000, vol. 356, pp. 1985-1986
NPL 2: C. L. Trimble and I. H. Frazer, Lancet Oncol., 2009, vol. 10, pp. 975-980
NPL 3: H. Poo et al., Int J. Cancer, 2006, vol. 119, pp. 1702-1709
NPL 4: N. G. Cortess-Perez et al., Vaccine, 2007, vol. 25, pp. 6581-6588
NPL 5: K. Adachi et al., Vaccine, 2010, vol. 28, pp. 2810-2817
NPL 6: H. Kiyohara et al., Evid. Based Complement. Alternat. Med., 2006, vol. 3, pp. 459-467
NPL 7: A. Dalpke et al., Immunology, 2001, vol. 204, pp. 667-676
NPL 8: D. Mizuno et al., Vaccine, 2011, vol. 29, pp. 5368-5378
NPL 9: H. Kayamuro et al., Biomaterials, 2009, vol. 30, pp. 5869-5876
NPL 10: H. Fujita et al., J. Immunol., 2009, vol. 183, pp. 254-260
NPL 11: Y. Morishima et al., Eur. J. Immunol., 2005, vol. 35, pp. 2803-2814
NPL 12: T. Matsumoto et al., Evidence-based complementary and alternative Medicine: ECAM, 2007, vol. 7, No. 1, pp. 69-77

### Summary of Invention

### Technical Problem

The present invention aims to solve the above existing problems and achieve the following object. That is, an object of the present invention is to provide an oral pharmaceutical composition or its use in a method for treating HPV infection which can be used for treatment of the cervical cancer, the vulvar cancer, the anal cancer, the oral cancer, the vaginal cancer, the penile cancer, the pharyngeal cancer, precursor lesions thereof, and the condyloma acuminatum, and which contains a HPV E7 polypeptide serving as a HPV vaccine and a Kampo preparation having a revitalizing activity.

### Solution to Problem

The present inventors conducted extensive studies to achieve the above object, and have found that administration of the HPV vaccine in combination with a mucosal adjuvant plus a Kampo preparation having a revitalizing activity (the Juzentaihoto and/or the Hochuekkito) enhances the HPV vaccine-specific cellular immunity on the mucosal region. Also disclosed is a mucosal immunity-stimulating agent containing a Kampo preparation having a revitalizing activity (e.g., the Juzentaihoto and the Hochuekkito) and a mucosal adjuvant, which is expected to function as not only a mucosal immunity-stimulating agent effective for a HPV vaccine but also a mucosal immunity-stimulating agent for other viral vaccines and bacterial vaccines.

The present invention is based on the above findings obtained by the present inventors. Means for solving the above problems are as follows.

A mucosal immunity-stimulating agent as also disclosed herein is characterized by containing a Kampo preparation having a revitalizing activity and a mucosal adjuvant.

An oral pharmaceutical composition of the present invention according to claim 5 or its use in a method for treating HPV infection according to claim 1 is characterized by containing a HPV E7 polypeptide, a mucosal adjuvant and a Kampo preparation having a revitalizing activity.

### Advantageous Effects of Invention

According to the present invention, these can solve the above existing problems and achieve the above object. That is, the present invention can provide an oral pharmaceutical composition according to claim 5 and its use in a method for treating HPV infection which can be used for treatment of the cervical cancer and precursor lesions thereof. The use in a method also encompasses a method of treating the vulvar cancer, the anal cancer, the oral cancer, the vaginal cancer, the penile cancer, the pharyngeal cancer, precursor lesions thereof, and the condyloma acuminatum.

Also disclosed is a mucosal immunity-stimulating agent which has a sufficient cellular immunity-stimulating effect on the mucosa, which can be administered in combination with a viral vaccine such as a HPV vaccine and a bacterial vaccine, and which contains a Kampo preparation having a revitalizing activity and a mucosal adjuvant.

### Brief Description of Drawings

FIG. 1 shows effects of oral coadministration of LacE7 with Juzentaihoto (JTT) or Hochuekkito (HET) on systemic and mucosal type 1 T cell responses.
FIG. 2 shows effects of oral coadministration of LacE7 with Juzentaihoto (JTT) or Hochuekkito (HET) plus LTB on systemic and mucosal type 1 T cell responses.
FIG. 3A shows effects of oral coadministration of LacE7 with Juzentaihoto (JTT) or Hochuekkito (HET) plus LTB on a systemic humoral immune response.
FIG. 3B shows effects of oral coadministration of LacE7 with Juzentaihoto (JTT) or Hochuekkito (HET) plus LTB on a mucosal humoral immune response.
FIG. 4A shows effects of oral coadministration of LacE7 with Juzentaihoto (JTT) or Hochuekkito (HET) plus LTB on a mucosal type 1 cell response.
FIG. 4B shows effects of oral coadministration of LacE7 with Juzentaihoto (JTT) or Hochuekkito (HET) plus LTB on a mucosal type 2 T cell response.
FIG. 5 shows effects of oral coadministration of LacE7 with Hochuekkito (HET) plus α-galactosylceramide (aGalCer) or LTB on a mucosal E7-specific type 1 T cell response.
FIG. 6 shows elicitation of an E7-specific CTL on the intestinal mucosal epithelium through oral administration of LacE7.

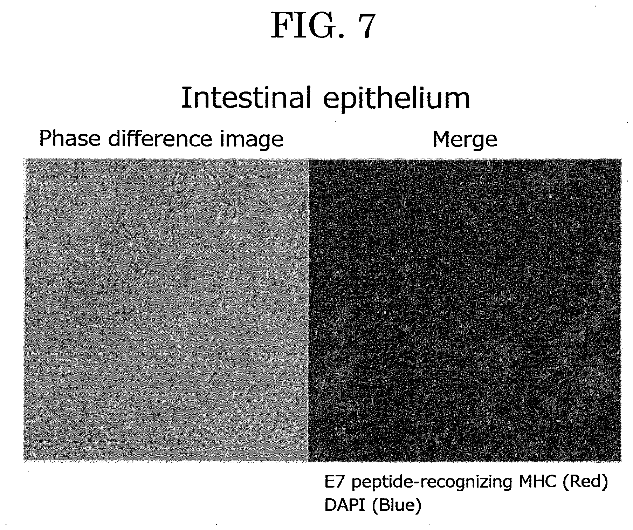

FIG. 7 shows elicitation of an E7-specific CTL on the intestinal mucosal epithelium through oral administration of LacE7.

### Description of Embodiments

### (Oral pharmaceutical composition)

The oral pharmaceutical composition of the present invention contains at least a Kampo preparation (immunity-enhancing Kampo preparation) having a revitalizing activity (immunity-enhancing effect), a HPV E7 polypeptide and a mucosal adjuvant; and, if necessary, further contains other appropriately selected ingredients.

### <Immunity-enhancing Kampo preparation>

The immunity-enhancing Kampo preparation having a revitalizing activity (immunity-enhancing effect) is Juzentaihoto and/or Hochuekkito. Juzentaihoto and/or Hochuekkito may be used alone or in combination with the other examples as additional components of the inventive oral pharmaceutical composition.

### <<Juzentaihoto>>

The Juzentaihoto is a kind of Kampo preparations, and consists of Astragalus Root; Ginseng; Cinnamon Bark; Japanese Angelica Root; Cnidium Rhizome; Peony Root; Rehmannia Root; Atractylodes Lancea Rhizome or Atractylodes Rhizome; Poria Sclerotium; and Glycyrrhiza. For example, the Juzentaihoto may contain 5.0 g of a mixed crude drug containing Japanese Pharmacopoeia (JP) Astragalus Root (3.0 g), JP Ginseng (3.0 g), JP Cinnamon Bark (3.0 g), JP Japanese Angelica Root (3.0 g), JP Cnidium Rhizome (3.0 g), JP Peony Root (3.0 g), JP Rehmannia Root (3.0 g), JP Atractylodes Lancea Rhizome (3.0 g), JP Poria Sclerotium (3.0 g), and JP Glycyrrhiza (1.5 g), relative to a total amount of 7.5 g. Furthermore, the Juzentaihoto may further contain, for example, JP Magnesium Stearate and JP Lactose Hydrate in addition to the mixed crude drug.

The Juzentaihoto may be commercially available products. Example of the commercially available products includes TSUMURA JUZENTAIHOTO EXTRACT GRANULES (Identification code: TSUMURA/48).

### <<Hochuekkito>>

The Hochuekkito is a kind of Kampo preparations, and consists of Astragalus Root; Ginseng; Atractylodes Lancea Rhizome or Atractylodes Rhizome; Japanese Angelica Root; Glycyrrhiza; Bupleurum Root; Citrus Unshiu Peel; Jujube; Ginger; and Cimicifuga Rhizome. For example, the Hochuekkito may contain 5.0 g of a mixed crude drug containing JP Astragalus Root (4.0 g), JP Ginseng (4.0 g), JP Atractylodes Lancea Rhizome (4.0 g), JP Japanese Angelica Root (3.0 g), JP Glycyrrhiza (1.5 g), JP Bupleurum Root (2.0 g), JP Citrus Unshiu Peel (2.0 g), JP Jujube (2.0 g), JP Ginger (0.5 g), and JP Cimicifuga Rhizome (1.0 g), relative to a total amount of 7.5 g. Furthermore, the Hochuekkito may further contain, for example, JP Magnesium Stearate and JP Lactose Hydrate in addition to the mixed crude drug.

The Hochuekkito may be commercially available products. Example of the commercially available products includes TSUMURA HOCHUEKKITO EXTRACT GRANULES (Identification code: TSUMURA/41).

### <Mucosal adjuvant>

The mucosal adjuvant is selected as defined by the claims. A synthetic ceramide of α-galactosylceramide (aGalCer) further enhances humoral immunity and cellular immunity which are specific for an oral vaccine (i.e., the HPV E7 polypeptide) cooperating with the Juzentaihoto or the Hochuekkito. Another example thereof include a bacterial toxin. αGalCer may be used alone or in combination with other examples as additional components.

An adjuvant derived from a bacterial toxin is preferable from the viewpoint of conventional and frequent usage as the mucosal adjuvant.

A synthetic ceramide of a-galactosylceramide (αGalCer) is used because it is not derived from a bacterial toxin. The synthetic ceramide of α-galactosylceramide (aGalCer) is used according to the invention, because it has been confirmed to have an adjuvant effect on human.

The bacterial toxin and the synthetic ceramide of α-galactosylceramide (aGalCer) have been known to have an adjuvant effect on a plurality of antigens.

For the oral pharmaceutical composition for treating HPV infection, the mucosal adjuvant is contained in the composition, because addition of the mucosal adjuvant can considerably enhance an effect of the oral pharmaceutical composition for treating HPV infection.

### <<Bacterial toxin-derived adjuvant>>

Also disclosed are bacterial toxin-derived adjuvants. Examples thereof include polypeptides derived from, for example, CT, LT, VT, DT, or PT They may be used alone or in combination as additional components of the inventive oral pharmaceutical composition.

Among them a mutant bacterial toxin-derived adjuvant is disclosed which has been mutated not to cause a severe side effect upon oral administration to human.

### -Mutant bacterial toxin-derived adjuvant-

Also disclosed is a mutant bacterial toxin-derived adjuvant as long as it does not cause a severe side effect upon oral administration to human. Examples thereof include a mutant CT, a mutant LT, a mutant VT, a mutant DT, and a mutant PT.

The mutant CT, the mutant LT, the mutant VT, the mutant DT, and the mutant PT are e.g. described in patent documents (WO 98/042375, WO 00/037609, WO 2006/100108, and WO 1996/006627). They may be used alone or in combination as additional components of the inventive oral pharmaceutical composition.

### --LTB polypeptide--

According to the invention, the LTB polypeptide is a polypeptide derived from a B subunit of the heat-labile enterotoxin form *Escherichia coli* LT (LTB) and has a mucosal adjuvant effect. Example thereof includes a full-length LTB protein.

The LTB exhibits toxicity in the form of a dimer with an A subunit of the LT (holotoxin), but does not exhibit toxicity alone.

Nevertheless, it has been found that the LTB protein acts as an adjuvant alone on the mucosa through oral or transnasal administration (WO 99/026654).

Therefore, the LTB polypeptide can also be used as the mucosal adjuvant in production of an oral vaccine for human use.

The LTB polypeptide may be commercially available products. Example of the commercially available products includes HEAT-LABILE ENTEROTOXIN, B SUBUNIT (LTB) FROM *E. COLI* (Catalog number: E8656) (manufactured by Sigma-Aldrich Corporation).

### <<Synthetic ceramide of α-galactosylceramide (aGalCer)>>

Sponge-derived ageliferins have been isolated through screening of naturally occurring compounds exhibiting an anti-tumor effect in an in vivo assay using mice with the biliary tract cancer. The synthetic ceramide of α-galactosylceramide (aGalCer) has been obtained as a derivative of the ageliferins which has a stronger anti-tumor effect. It has been found that the synthetic ceramide of α-galactosylceramide (aGalCer) acts as a so-called "adjuvant" from the result in a subsequent study that, after uptake into an antigen-presenting cell, the synthetic ceramide of α-galactosylceramide elicits production of IFN-γ and IL-4 through association with a NKT cell which share properties of both of a NK cell and a T cell. The synthetic ceramide of α-galactosylceramide also acts as the mucosal adjuvant because it has been reported to be capable of inducing production of IgA in the nose through transnasal administration.

The synthetic ceramide of α-galactosylceramide (aGalCer) may be commercially available products. Example of the commercially available products includes α-galactosylceramide (KRN7000) (manufactured by Kyowa Hakko Kirin Co., Ltd.).

### <Other ingredients>

The other ingredients are not particularly limited and may be appropriately selected depending on the intended purpose, as long as they do not impair effects of the present invention. Example thereof includes a pharmaceutically acceptable carrier. Amounts of the other ingredients contained in the pharmaceutical composition are not particularly limited and may be appropriately selected depending on the intended purpose.

### <Application>

It is disclosed for the mucosal immunity-stimulating agent that it can be orally administered to a patient. In this case, the mucosal immunity-stimulating agent stimulates cellular immunity specific for an antigen administered before, after, or concurrently with it on the mucosa of the patient.

### <<Antigen>>

Generally, the antigen administered before, after, or concurrently with the mucosal immunity-stimulating agent is not particularly limited, as long as it is a peptide derived from a virus or a bacterium and capable of being used as a vaccine. The antigen may be used alone or in combination. The therapeutic use may be against a HPV-associated cancer (e.g., cervical cancer). According to the invention, the antigen is a polypeptide derived from HPVE7.

### -HPV-derived polypeptide-

The HPV-derived polypeptide is not particularly limited and may be appropriately selected depending on the intended purpose, as long as it exhibits antigenicity. For example, the HPV-derived polypeptide may be a full-length HPV-derived protein. The HPV-derived polypeptide may have a mutation such as deletion, substitution, or addition of one to several amino acids. The HPV E7-derived polypeptide may be used alone or in combination with other HPV-derived polypeptides.

### --HPV E7 polypeptide--

The HPV-derived polypeptide is a HPV E7 polypeptide according to the invention. The HPV E7 polypeptide is not particularly limited and may be appropriately selected depending on the intended purpose. More preferable are, for example, those derived from E7 of at least one HPV selected from the group consisting of HPV16, HPV18, HPV31, HPV33, HPV35, HPV39, HPV45, HPV51, HPV52, HPV56, HPV58, HPV59, HPV68, HPV73, HPV82, HPV26, HPV53, and HPV63 which belong to a high-risk group for cancer.

Among them, particularly preferable are those derived from E7 of HPV16, HPV18, or both thereof, and most preferable are those derived from HPV16 E7.

The HPV E7 polypeptide may be any polypeptide as long as it has antigenicity. Example thereof includes a full-length E7 protein.

The E7 polypeptide may be derived from a wild-type E7 or may be a mutant E7 polypeptide derived from a mutant E7.

### ---Mutant E7 polypeptide---

The mutant E7 polypeptide is not particularly limited and may be appropriately selected depending on the intended purpose. Examples thereof include those lacking a binding property with an Rb protein through deletion, substitution, or addition of one to several amino acids. The mutant E7 polypeptide may be used alone or in combination.

Among these mutant E7 polypeptides, preferable are those in which amino acid residues of the E7 protein involved in binding with the Rb protein (i.e., Asp at position 21, Cys at position 24, and Glu at position 26 of a wild-type HPV16 E7 protein (SEQ ID No. 1)) are substituted with other amino acid residues. For example, the mutant HPV16 E7 protein (SEQ ID No. 2) in which Asp at position 21, Cys at position 24, and Glu at position 26 of the wild-type HPV16 E7 protein (SEQ ID No. 1) are all substituted with Gly may be used.

A mucosal immunity-stimulating agent as disclosed herein enhances acquired immunity, in particular, cellular immunity specific for a vaccine, in the case where it is orally administered together with the vaccine (e.g., a viral antigen or a bacterial antigen orally administered as an immunogen).

The HPV-derived polypeptide is added to a powder of killed *Lactobacillus casei,* or a powder of killed recombinant *Lactobacillus casei* expressing the HPV E7 polypeptide.

A method for producing the HPV-derived polypeptide intended for being used as a vaccine is also disclosed. It is not particularly limited and may be appropriately selected depending on the intended purpose. Examples thereof include a production method using a chemical synthesis or a genetic recombination technology.

Among them a production method using a genetic recombination in a transformed bacterium from the viewpoint of cost is disclosed herein.

The bacterium used as a host is not particularly limited and may be appropriately selected depending on the intended purpose. Examples thereof include yeast, *E. coli, Bacillus subtilis,* and a lactic acid bacterium. Among them, preferable is the lactic acid bacterium, more preferable is *Lactobacillus spp.,* and particularly preferable is *Lactobacillus casei* because it has been confirmed to induce a type 1 helper T cell (Th1).

An expression vector suitable for expressing in the host bacterium may be appropriately selected.

An expression vector containing nucleic acids coding for the HPV-derived polypeptide (expression vector for HPV-derived polypeptide) can be used to transform the host bacterium through a transformation method appropriately selected depending on the intended purpose.

The HPV-derived polypeptide may express on or in cells of the host bacterium or be extracellularly secreted through appropriate selection of the expression vector.

A method for culturing a host bacterium transformed with the expression vector for HPV-derived polypeptide (HPV-derived polypeptide-expressing host bacterium) is also disclosed. It is not particularly limited and may be appropriately selected depending on the intended purpose.

The HPV-derived polypeptide may be purified from a culture of the HPV-derived polypeptide-expressing host bacterium and used as a vaccine. Alternatively, the HPV-derived polypeptide-expressing host bacterium itself may be used as a vaccine.

### (Oral pharmaceutical composition or its use in a method for treating HPV infection)

An oral pharmaceutical composition for treating HPV infection of the present invention contains at least a HPV E7 polypeptide, a mucosal adjuvant and a Kampo preparation having a revitalizing activity (i.e., immunity-enhancing effect) (immunity-enhancing Kampo preparation); and, if necessary, further contains other appropriately selected ingredients.

A method for producing the E7 polypeptide intended for being used as a vaccine is not particularly limited and may be appropriately selected depending on the intended purpose. Examples thereof include a production method using a chemical synthesis or a genetic recombination technology.

Among them, preferable is a production method using a genetic recombination in a transformed bacterium from the viewpoint of cost.

The bacterium used as a host is not particularly limited and may be appropriately selected depending on the intended purpose. Examples thereof include yeast, *E. coli, Bacillus subtilis,* and a lactic acid bacterium. Among them, preferable is the lactic acid bacterium, more preferable is *Lactobacillus spp.,* and particularly preferable is *Lactobacillus casei* because it has been confirmed to induce a type 1 helper T cell (Th1).

An expression vector suitable for expressing in the host bacterium may be appropriately selected.

An expression vector containing nucleic acids coding for the E7 polypeptide (expression vector for E7 polypeptide) can be used to transform the host bacterium through a transformation method appropriately selected depending on the intended purpose.

The E7 polypeptide may express on or in cells of the host bacterium or be extracellularly secreted through appropriate selection of the expression vector.

A method for culturing a host bacterium transformed with the expression vector for E7 polypeptide (E7 polypeptide-expressing host bacterium) is not particularly limited and may be appropriately selected depending on the intended purpose.

The E7 polypeptide may be purified from a culture of the E7 polypeptide-expressing host bacterium and used as a vaccine. Alternatively, the E7 polypeptide-expressing host bacterium itself may be used as a vaccine.

In the case where the E7 polypeptide-expressing host bacterium itself is used as a vaccine, it may be killed by, for example, heating depending on the intended purpose to thereby obtain a killed E7 polypeptide-expressing host bacterium.

The killed E7 polypeptide-expressing host bacterium may be collected from a medium, followed by drying to powder to thereby obtain a dried powder product, which may be used as a vaccine.

The dried powder product may be stored at a low temperature until use.

The E7 polypeptide is added to a powder of killed *Lactobacillus casei,* and the resultant may be used as a vaccine.

### <<Carrier>>

The carrier is not particularly limited and may be appropriately selected depending on, for example, a dosage form of the pharmaceutical composition described below.

### -Dosage form-

The dosage form is not particularly limited, as long as it can be orally administered. Examples thereof include an oral solid preparation (e.g., a tablet, a coated tablet, a granule, a powder, and a capsule) and an oral solution (e.g., an internal liquid, a syrup, and an elixir).

### <Application>

The oral pharmaceutical composition for use in a method of treating HPV infection can be used for stimulating cellular immunity specific for a HPV E7 polypeptide (HPV vaccine) on the mucosa of a patient (e.g., a patient with a cervical cancer precursor lesion and a patient with the cervical cancer).

### Examples

Examples of the present invention will be described below, but the present invention is not limited thereto.

### (Example 1)

### <Immunization of mouse for in vivo assay>

Mice were orally immunized with a LacE7 preparation according to the procedure described in K. Adachi et al., Vaccine, 2010, vol. 28, pp. 2810-2817. Upon immunization, the mice were orally administered with Juzentaihoto (hereinafter referred to as "JTT") and Hochuekkito (hereinafter referred to as "HET"), if necessary, and, further orally administered with a LTB polypeptide or a synthetic ceramide (aGalCer), if necessary.

### <<Production of LacE7 preparation>>

The LacE7 preparation was produced according to the known method (Poo H. et al., Int. J. Immunol., 2006, vol. 119, pp. 1702-1709). Briefly, a mutant E7 protein derived from HPV16 which had the amino acid sequence represented by SEQ ID No. 2, that is, in which three amino acid residues at a Rb binding site of a wild-type E7 protein were substituted was introduced via an expression vector into *Lactobacillus casei* which had been confirmed to induce a type 1 helper T cell (Th1 cell). The resultant recombinant (LacE7) was cultured in a medium, followed by heat-killing. The LacE7 was purified from the medium, washed with distilled water several times, and dried to powder (LacE7 preparation). The resultant preparation was stored at 4°C until use. The LacE7 preparation was insoluble in an aqueous solvent.

### <<Oral administration of LacE7 preparation to mouse>>

Six-week old female SPF C67BL mice (manufactured by CLEA Japan, Inc.) were immunized with the LacE7 preparation through oral administration.

The LacE7 preparation (1.0 mg per mouse) was administered for a total of 4 rounds at weeks 1, 2, 4, and 6. For all administration, the LacE7 preparation was suspended in PBS (200 µL) and administered via an intra-gastric tube after 3 hours of fasting once per day for consecutive five days every week.

### <<Oral administration of Juzentaihoto and Hochuekkito to mouse>>

JTT (manufactured by TSUMURA & CO.) or HET (manufactured by TSUMURA & CO.) (40 mg/mouse/day) mixed with powdered diet (5 g) was consumed completely by 5 mice in a single cage. The JTT or the HET was administered to the mice every day for 6 weeks (weeks 1 to 6) during administration of the LacE7 preparation.

### <<Oral administration of LTB polypeptide to mouse>>

The LTB polypeptide (10 µg/mouse) was orally administered together with the LacE7 preparation on the third day of each round. The LTB polypeptide used was HEAT-LABILE ENTEROTOXIN, B SUBUNIT (LTB) FROM *E. COLI* (catalog number: E8656) (manufactured by Sigma-Aldrich Corporation).

### <<Oral administration of aGalCer to mouse>>

The synthetic ceramide aGalCer (2 µg/mouse) was orally administered together with the LacE7 preparation on the third day of each round. The synthetic ceramide used was α-galactosylceramide (KRN7000) (manufactured by Kyowa Hakko Kirin Co., Ltd.).

### <Collection of spleen, intestinal lavage fluid, peripheral blood sample, and mucosal tissue>

Lymphocytes, sera, and intestinal lavage fluid were collected one week after the last inoculation of the LacE7 preparation (at week 7).

The five mice to which the LacE7 preparation had been inoculated were dissected, from which spleens, intestines, and peripheral bloods were collected. The spleens were washed with HBSS (Hanks' Balanced Salt Solution). For the intestines, after faces removal, the inside of each of the intestinal tracts was washed with HBSS (10 mL) supplemented with a protease inhibitor. The collected fluid was used as intestinal lavage fluid. The collected sera and the intestinal lavage fluid were stored at -80°C until use.

Similarly, the twenty five mice to which the LacE7 preparation had been inoculated were dissected, from which the intestinal epitheliums were collected as mucosal tissues. The intestinal epitheliums were fixed with phosphate buffered saline (PBS) containing 4% paraformaldehyde at room temperature for 1 hour, followed by rinsing with PBS. Each of the resultants was embedded in OCT compound (TISSUE-TEK (registered trademark), manufactured by Sakura Finetek, Zoeterwoude, The Netherlands), followed by freezing and storing at -80°C. Frozen sections (thickness: 6 µm) were cut on a cryostat (CM1510S, manufactured by Leica) and placed onto a glass slide for an immunohistochemical analysis.

### <Preparation of murine splenocyte and mucosa lymphocyte>

Each intestine was opened longitudinally and shaken vigorously in a PRMI1640 medium, which contained 10% by mass inactivated fetal bovine serum, 2 mM L-glutamine, 1 mM sodium private, 1 × non-essential amino acids, and 50 mM 2-mercaptoethanol, supplemented with 10% by mass FBS, 100 unit/mL of penicillin and 100 µg/mL of streptomycin at 37°C for 30 min. Cell suspensions were collected therefrom, passed through BD FALCON (trademark) Cell strainer (manufactured by BD Bioscience, USA) to thereby remove tissue debris and then were subjected to discontinuous density gradient centrifugation in a 15 mL tube layered from the bottom with 70% by mass and 40% by mass of PERCOLL PLUS. About 10⁷ to about 10⁸ cells were layered thereon, followed by centrifugation at 600 × g and room temperature for 20 min. As a result, mucosal lymphocytes (cell viability: 95%) were concentrated at the interface between the 70% by mass PERCOLL PLUS layer and the 40% by mass PERCOLL PLUS layer contained.

Splenocytes were prepared by gently teasing the spleen in PBS. Clumped debris was removed by centrifugation.

About 5 × 10⁶ to about 10 × 10⁶ mucosa lymphocytes and about 10⁷ splenocytes were obtained from each mouse.

### <ELISPOT assay>

A suspension (50 µL) of the intestinal mucosal lymphocytes or the splenocytes (5 × 10⁶ cells/mL) was incubated for 24 hour at 37°C together with antigen presenting cells. Fifty microliters of the splenocytes (5 × 10⁶ cells/mL) were treated with mitomycin, followed by washing 3 times with PBS. According to a manufacture's instruction of MOUSE IFN-γ ELISPOT KIT (manufactured by MABTECH AB, Sweden), 10 µL of a synthetic peptide (1 µg/mL) consisting of amino acids at positions 49 to 57 of E7 protein which had reported to be a CTL epitope for HPV16 E7 protein (SEQ ID No. 1), mitogen (40 ng/mL of phorbol myristate acetate plus 4 µg/mL of ionomycin), or a medium alone (control) were added dropwise to a 96-well plate (for example, ELIIP plate, manufactured by Millipore, USA) coated with anti-mouse IFN-γ antibodies,. The number of IFN-γ-positive spots on the 96-well plate was analyzed with a fully automated computer assisted video imaging analysis system, KS ELISPOT (manufactured by Carl Zeiss Vision, Germany).

### <ELISA assay>

IgGs in the sera and IgAs in the intestinal lavage fluid were quantified with MOUSE IgG ELISA QUANTITATION SET or MOUSE IgA ELISA QUANTITATION SET (manufactured by Bethyl Laboratories, Inc., USA). Amounts of HPV16 E7 protein-specific IgGs and IgAs were quantified using a His-tagged E7 protein as an antigen, according to a manufacture's instruction.

Briefly, a 96-well plate (e.g., IMMUNOPLATE, Nunc, Denmark) was coated with 100 ng/well of the His-tagged E7 which was suspended in a coating buffer containing 50 mM carbonate-bicarbonate (pH 9.6) overnight at 4°C. Thereafter, the plate was blocked and doubling diluted-sera and intestinal lavage fluid were applied thereto. After washing, peroxidase-labeled anti-mouse IgG antibodies or anti-mouse IgA antibodies (diluted at 1:60,000 and 1:20,000, respectively) serving as secondary antibodies were added to each well, followed by incubating at 37°C for 1 hour. After washing and developing, the plate was measured for absorbance at 450 nm. Serum and intestinal lavage fluid of a non-immunized mouse were used as a negative control. A titer of an anti-E7 antibody was represented as anti-E7 IgG/total IgG or anti-E7 IgA/total IgA (relative value).

### <Measurement of cytokine concentration>

The intestinal lavage fluid obtained from the 5 mice were pooled and measured for cytokine concentrations using MOUSE TH1/TH2 ELISA READY SET GO (registered trademark) kit (BD Bioscience, USA). In this measurement, IFN-γ and IL-4 were used a Th1 cell-expressing cytokine and a Th2 cell-expressing cytokine, respectively. Measurements were repeated at least three times for each intestinal lavage fluid.

### <Statistical analysis>

ELISPOT and ELISA data were represented as means ± standard deviations. These values or relative values were compared between immunization groups (5 mice/group) using a two-sided Student's t-test. A p-value of <0.05 was considered to be significant.

### <Immunohistochemical analysis>

Induction and infiltration into the mucosal tissue of E7 protein-specific cytotoxic T cells (E7-specific CTLs) were examined with T-SELECT MOUSE MHC TETRAMER (manufactured by MEDICAL & BIOLOGICAL LABORATORIES CO., LTD.) in the mice to which the LacE7 had been orally administered. AMCH-E7 peptide complex was biotinylated and tetramerized with phycoerythrin-labeled streptavidin according to a manufacture's instruction to thereby produce an E7-specific MHC tetramer. The synthetic peptide (1 µg/mL) consisting of amino acids at positions 49 to 57 of E7 protein which had reported to be a CTL epitope for HPV16 E7 protein (SEQ ID No. 1) was used as an E7 peptide.

As mentioned above, intestinal epidermal sections were obtained from the mice to which the LacE7 had been orally administered, followed by subjecting to detection of an E7 peptide-recognizing MHC with the E7-specific MHC tetramer, or double staining with the E7-specific MHC tetramer and anti-CD8 antibody. Specifically, each of the intestinal epidermal section samples was permeabilized with PBS containing 0.1% by mass TRITON-X100 for 30 min. Thereafter, the sample was rinsed and impregnated with PBS containing 5% by mass of skimmed milk for 30 min. The resultant was incubated overnight at 4°C with the E7-specific MHC tetramer (dilution rate: 1:25) and optionally the anti-CD8 antibody (RAT ANTI-MOUSE CD8A/LYT-2 FITC (Clone 53-6.7), manufactured by Beckman Coulter, Inc.) diluted at 1:50. For each step, the E7-specific MHC tetramer and the antibody were diluted with PBS containing 1% by mass of skimmed milk and 0.1% by mass of TRITON-X100. The resultant section was subjected to nuclear staining with DAPI (4',6-Diamidine-2-phenylindole dihydrochloride, manufactured by Roche), followed by placing into PBS/glycerol (1/1) and observing by a fluorescence microscope (LSM 700, manufactured by Zeiss).

### <Adjuvant effect of Kampo preparation on E7-specific 1 type T cell response>

For clearance of cells which have been immortalized by expression of HPV E6 protein and E7 protein, an E7-specific T cell response (hereinafter referred to as "type 1 T cell response") by an IFN-γ-producing CD4⁺ helper T cell and a HPV E7 protein-recognizing CD8⁺ CTL is necessary.

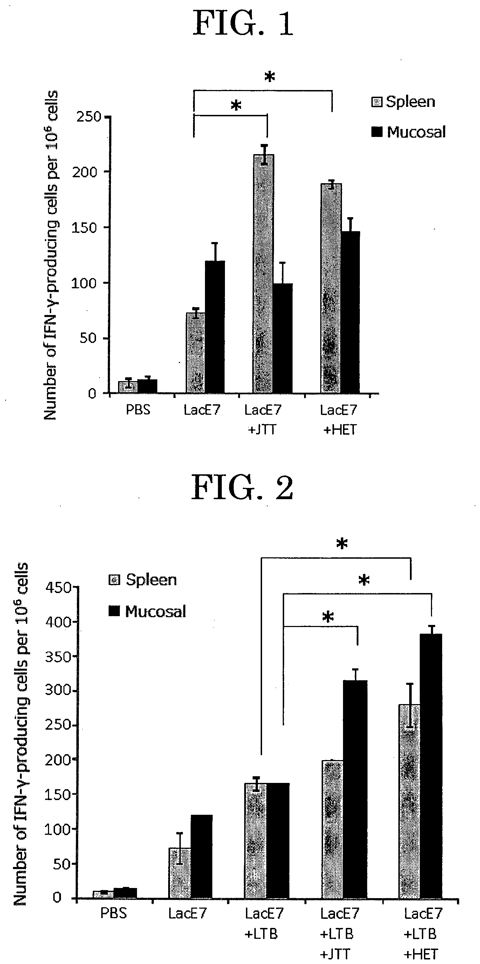

To examine the effect of oral administration of a LacE7 vaccine with a Kampo preparation on the E7-specific 1 type T cell response, the numbers of IFNy-producing cells in the mucosal lymphocytes and the splenocytes were assessed by ELISPOT assay (FIG. 1).

Five mice per group were orally administered with the LacE7 alone, or the LacE7 plus the JTT or the HET. Intestinal mucosal lymphocytes and splenocytes were collected from each mouse one week after the last immunization to thereby examine the effect of the JTT or the HET on mucosal and systemic immunities.

Amino acid residues at positions 49 to 57 of HPV16 E7 have been known to be an E7-CTL epitope which is recognized by CD4⁺ T cells and CD8⁺ T cells of C57BL/6 mice. Therefore, a synthetic peptide consisting of amino acids corresponding to the E7-CTL epitope site was used in the ELISPOT assay for the type 1 T cell response against the HPV16 E7 protein.

The numbers of E7-specific IFN-γ-producing cells in both the mucosal lymphocytes and the splenocytes were increased in LacE7-immunized mice compared to non-immunized mice. Oral immunization with the LacE7 increased a level of the E7-specific IFN-γ-producing cells in the mucosal lymphocytes more considerably (about 1.5-fold to about 2-fold higher) than in the splenocytes.

Further surprisingly, coadministration of the LacE7 with the JTT or the HET considerably improved the E7-specific type 1 T cell response in the splenocytes (FIG. 1).

The JTT and the HET have been suggested to have a humoral immunity-enhancing effect through the use as an adjuvant for a prophylactic vaccine.

However, the JTT and the HET have not been known to enhance a systemic cellular immunity, which is a new finding of the present inventors.

Meanwhile, regarding the type 1 T cell response in the mucosa, the JTT or the HET did not exhibit a considerable effect in an oral coadministration with the LacE7 (FIG. 1).

### <Adjuvant effect of Kampo preparation and LBT polypeptide on mucosal immune response>

The present inventors thought that use of another adjuvant other than the JTT or the HET was needed to be examined in order to optimize the mucosal cellular immunoresponsiveness to the E7 protein.

Therefore, the LTB polypeptide was coadministered with the JTT or the HET during oral administration of the LacE7.

Coadministration of the LacE7 and the LTB polypeptide increased a level of the E7-specific type 1 T cell response in mice 1.5-fold to 2-fold higher than administration of the LacE7 alone both in the mucosal lymphocytes and the splenocytes (FIG. 2). Coadministration of the JTT or the HET with the LacE7 plus the LTB polypeptide increased a systemic level of the type 1 T cell response compared to coadministration of the LacE7 with the LTB polypeptide, which effect was particularly considerable in the HET.

Further, as expected, through additional coadministration of the JTT or the HET, a mucosal level of the type 1 T cell response was also increased 2-fold to 2.5-fold higher than coadministration of the LacE7 with the LTB (FIG. 2).

That is, it was found that coadministration of the JTT or the HET with the LTB considerably increased mucosal and systemic levels of the type 1 T cell response by the action of an oral vaccine.

### <Humoral immune response-enhancing effect against E7 of Kampo preparation adjuvant>

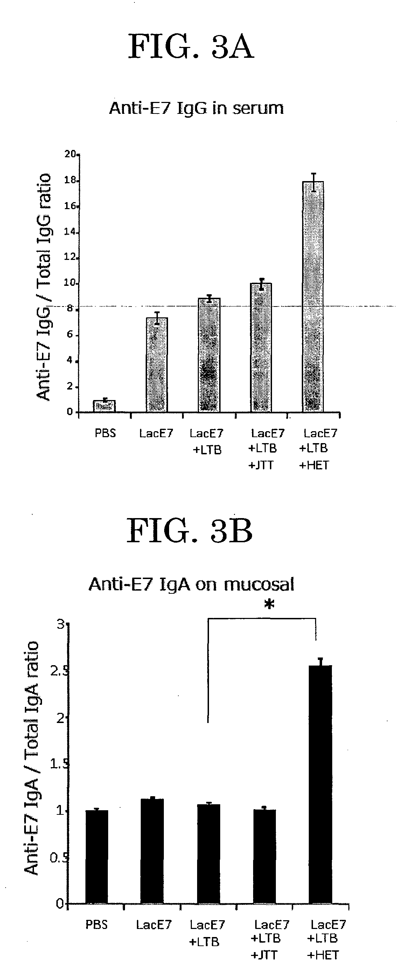

Next, an effect of coadministration of the Kampo preparation, the LTB, or both thereof on a humoral immune response against HPC16 E7 in the mice to which the LacE7 had been orally administered was examined (FIGs. 3A and 3B). From the five mice per group which had been orally administered with the LacE7 alone or the LacE7 plus the JTT or the HET and then from which the intestinal mucosa lymphocytes and the splenocytes had been collected one week after the last immunization, blood and intestinal lavage fluid were also collected one week after the last immunization. Regarding systemic and mucosal humoral immunities elicited by the oral administration of the LacE7, amounts of anti-E7 IgG antibodies in the sera and anti-E7 IgA antibodies in the intestinal lavage fluid were measured with a sandwich ELISA method (FIGs. 3A and 3B). To avoid bias due to sampling, titers of anti-E7 antibodies are represented as relative values relative to a total IgG amount and a total IgA amount in the sample.

A ratio of an amount of the anti-E7 antibody to the total IgG amount or the total IgA amount (anti-E7 IgG/total IgG or anti-E7 IgA/total IgA) represents a systemic or mucosal humoral immune response against E7 protein, respectively. The ratio in the mouse which had not been immunized with the LacE7 was determined as 1, and the ratio thereto was reported.

In the mice to which the LacE7 alone had been orally administered, the titer of the anti-E7 IgG antibody in sera was increased 7-fold or more. In the case where the LTB was coadministered with the LacE7, the LTB had almost no effect on the titer of the anti-E7 IgG antibody.

Meanwhile, in the mice to which the HET had been coadministered with the LacE7 plus the LTB, the titer of the anti-E7 IgG antibody in sera was increased 2-fold or more higher than the mice to which the LacE7 alone had been administered or the LacE7 had been coadministered with the LTB.

In contrast, coadministration of the JTT with the LacE7 plus the LTB had almost no effect on the titer of the anti-E7 IgG antibody (FIG. 3A).

In the case where the HET was coadministered with the LacE7 plus the LTB, the titer of the anti-E7 IgA antibody was also considerably increased in the intestinal lavage fluid (FIG. 3B).

These results strongly suggest that the HET and the LBT synergistically enhance systemic and mucosal humoral immunities elicited by the oral vaccine.

### <Effect of Kampo adjuvant on mucosal cytokine production by LacE7>

Productions of a cytokine which promotes the type 1 T cell response and a cytokine which promotes a cell response by Th2 cells (hereinafter referred to as "type 2 T cell response") in a mucosal tissue induced by orally coadministration of the Kampo preparation with the LacE7 were examined.

Levels of IFN-γ and IL-4 in the intestinal lavage fluid from mice to which the LacE7 had been orally administered were indicative of the type 1 T cell response and the type 2 T cell response, respectively.

In the mice which had been immunized with the LacE7, amounts of the IFN-γ and the IL-4 in the intestinal lavage fluid were considerably increased compared to the non-immunized mouse. In the case of coadministration of the LacE7 with the JTT, amounts of the IFN-γ and the IL-4 were increased 3-fold higher. Meanwhile, there was no considerable difference between the case of coadministration of the LacE7 with the HET or the LTB and the case of administration of the LacE7 alone.

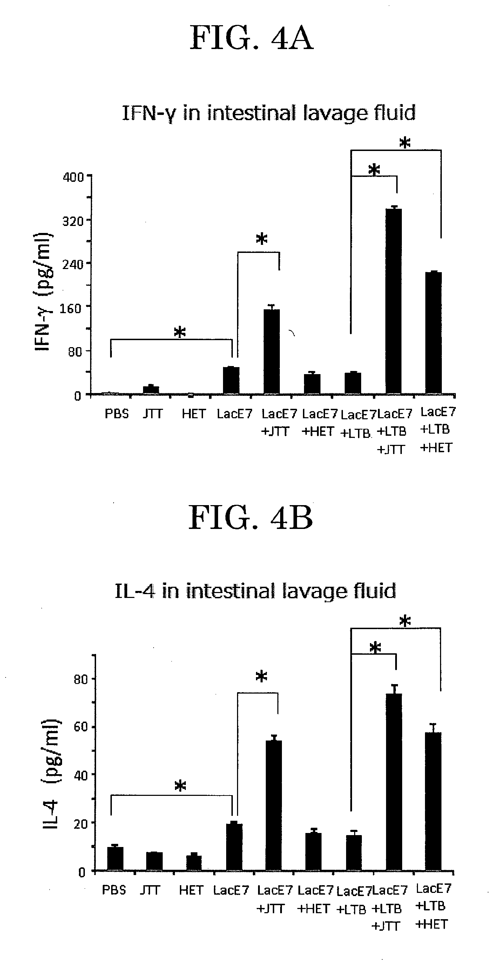

In the case of coadministration of the LTB with the LacE7 plus the JTT or the HET, an amount of the IFN-γ was increased to 6-fold to 8-fold, and an amount of the IL-4 was increased to 3-fold to 4-fold (FIGs. 4A and 4B).

That is, using cytokine production as an index, it has been found that the JTT has a stronger effect on the type 1 T cell response in the mucosa elicited by the oral vaccine than the HET, that effects of the JTT or the HET were further enhanced by using in combination with the LTB, and that the type 1 T cell response had the similar tendency to the type 2 T cell response.

The type 1 T cell response and the type 2 T cell responses elicit the cellular immunity and the humoral immunity in the spleen, respectively, there was no considerable correlation between elicitation effects of the cellular immunity and the humoral immunity by the JTT and the HET, and the type 1 T cell response and the type 2 T cell response in the mucosa.

This suggests a possibility that, in the mucosa, not only Th1 and Th2, but also other T cells such as Th17 are responsible for elicitation of the cellular immunity and the humoral immunity.

### <Adjuvant effect of Kampo preparation and synthetic ceramide on mucosal immune reaction>

It was examined whether the type 1 T cell response was increased in the mucosa in the case where aGalCer, which was a clinically available mucosal adjuvant, was used as a mucosal adjuvant in coadministration of the E7 protein, the Kampo preparation, and the mucosal adjuvant.

Therefore, αGalCer was coadministered with the JTT instead of the LTB polypeptide during the oral administration of the LacE7. The level of the E7-specific type 1 T cell response was examined with the ELISPOT assay.

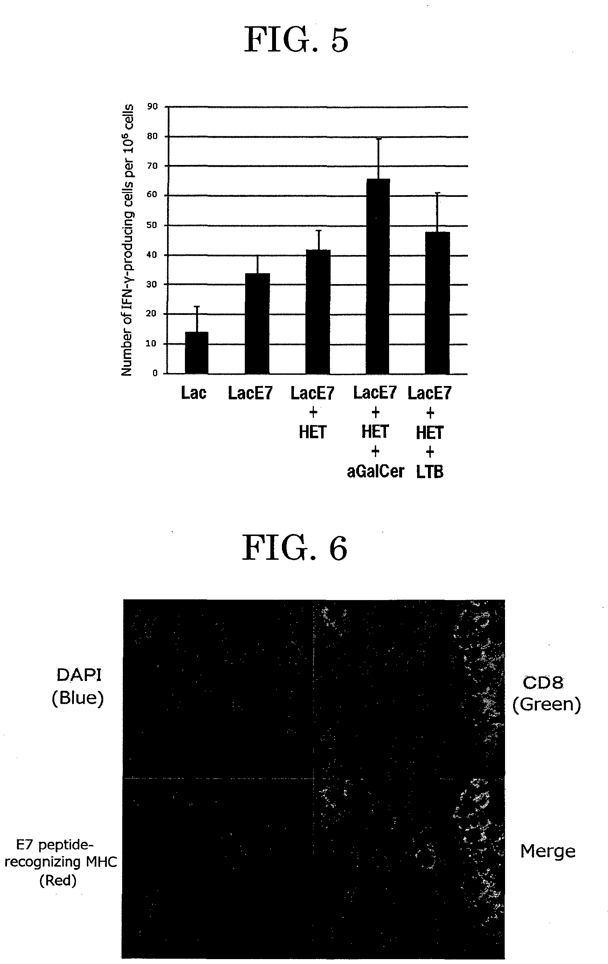

As can be seen from FIG. 2, the coadministration of the LacE7 with the LTB polypeptide increased the level of the E7-specific type 1 T cell response in murine mucosal lymphocytes to a greater extent than the administration of the LacE7 alone. Furthermore, the tendency was observed that the coadministration of the HET with the LacE7 plus the LTB polypeptide increased the level to a greater extent than the coadministration of the LacE7 with the LTB polypeptide (FIG. 5).

In the case where the αGalCer was used as the mucosal adjuvant instead of the HET in the coadministration of the E7 protein, the Kampo preparation, and the mucosal adjuvant, the level of the E7-specific type 1 T cell response in murine mucosal lymphocytes was also considerably increased to the same extent as or to a greater extent than the case of using the HET (FIG. 5). Note that, the coadministration of the LacE7 with the αGalCer increased the level to the same extent as the administration of the LacE7 (data not shown).

That is, it has been found that, through coadministration with the LTB, the αGalCer considerably increases the type 1 T cell response in the mucosa by the action of an oral vaccine.

### <Induction and infiltration into mucosal tissue of E7-specific CTL by LacE7>

Induction of the E7 protein-specific cytotoxic T cell (E7-specific CTL) in the mucosal tissue of the mice to which the LacE7 had been administered was examined with the immunohistochemical analysis. As a result, the E7 peptide-specific MHC was detected on a mucosa side and lamina propria mucosae in an intestinal epithelium which was the mucosal tissue. Therefore, the induction of the E7-specific CTL was recognized and infiltration of the E7-specific CTL into the mucosal tissue was revealed.

This result indicates that the oral administration of the LacE7 elicits the E7-specific mucosal immunity.

From the above results, a LacE7 vaccine containing either Juzentaihoto or Hochuekkito can be well expected to have a therapeutic effect against not only a CIN3 patient, but also a cervical cancer patient, other HPV-associated cancer patients and a patient with precursor lesion of the HPV-associated cancer.

A LacE7 vaccine containing an adequate immune adjuvant in addition to Juzentaihoto or Hochuekkito can be expected to have a more excellent therapeutic effect.

### Industrial Applicability

A mucosal immunity-stimulating agent as disclosed herein is suitable for stimulating a mucosal immunity by using in combination with a viral vaccine (e.g., HPV vaccine) or a bacterial vaccine.

An oral pharmaceutical composition or its use in a method for treating HPV infection of the present invention is suitable for using as a therapeutic vaccine against HPV infection by administering to a patient suffering from the HPV infection.

## Claims

1. An oral pharmaceutical composition for use in a method of treating human papillomavirus (HPV) infection which stimulates cellular immunity specific for a HPV E7 polypeptide in a mucosa of a patient, comprising:
a HPV E7 polypeptide;
a Kampo preparation having a revitalizing activity; and
a mucosal adjuvant,
wherein the Kampo preparation having a revitalizing activity is Juzentaihoto, Hochuekkito, or both thereof,
wherein the mucosal adjuvant is a polypeptide derived from the B subunit of the heat-labile enterotoxin from *Escherichia coli* or the synthetic ceramide of α-galactosylceramide, and
wherein the HPV E7 polypeptide is contained in a powder of killed recombinant *Lactobacillus casei* expressing the HPV E7 polypeptide or is added to a powder of killed *Lactobacillus casei.*

2. The oral pharmaceutical composition for use in a method of treating HPV infection according to claim 1, wherein the Kampo preparation having a revitalizing activity is Juzentaihoto.

3. The oral pharmaceutical composition for use in a method of treating HPV infection according to any one of claims 1 to 2, wherein the HPV E7 polypeptide is derived from HPV16.

4. The oral pharmaceutical composition for use in a method of treating HPV infection according to any one of claims 1 to 3, wherein the patient is a patient with a cervical cancer precursor lesion or a cervical cancer patient.

5. An oral pharmaceutical composition suitable for treating HPV infection which stimulates cellular immunity specific for a HPV E7 polypeptide in a mucosa of a patient, comprising:
a HPV E7 polypeptide;
a Kampo preparation having a revitalizing activity; and
a mucosal adjuvant,
wherein the Kampo preparation having a revitalizing activity is Juzentaihoto, Hochuekkito, or both thereof,
wherein the mucosal adjuvant is a polypeptide derived from the B subunit of the heat-labile enterotoxin from *Escherichia coli* or the synthetic ceramide of α-galactosylceramide, and
wherein the HPV E7 polypeptide is contained in a powder of killed recombinant *Lactobacillus casei* expressing the HPV E7 polypeptide or is added to a powder of killed *Lactobacillus casei.*

6. The oral pharmaceutical composition suitable for treating HPV infection according to claim 5, wherein the Kampo preparation having a revitalizing activity is Juzentaihoto.

7. The oral pharmaceutical composition suitable for treating HPV infection according to any one of claims 5 to 6, wherein the HPV E7 polypeptide is derived from HPV16.

8. The oral pharmaceutical composition suitable for treating HPV infection according to any one of claims 5 to 7, wherein the patient is a patient with a cervical cancer precursor lesion or a cervical cancer patient.

## Patentansprüche

1. Eine orale pharmazeutische Zusammensetzung zur Verwendung in einem Verfahren zur Behandlung von humanen Papillomavirus (HPV)-Infektionen, die die zelluläre Immunität stimuliert, die spezifisch für ein HPV E7-Polypeptid in einer Mucosa eines Patienten ist, umfassend:
ein HPV-E7-Polypeptid;
ein Kampo-Präparat mit einer revitalisierenden Wirkung; und
ein Schleimhautadjuvans,
wobei das Kampo-Präparat mit einer revitalisierenden Aktivität Juzentaihoto, Hochuekkito oder beides ist,
wobei das Schleimhautadjuvans ein Polypeptid ist, das von der B-Untereinheit des hitzelabilen Enterotoxins aus *Escherichia coli* oder dem synthetischen Ceramid von α-Galactosylceramid abgeleitet ist, und
wobei das HPV-E7-Polypeptid in einem Pulver aus abgetötetem rekombinantem *Lactobacillus casei,* der das HPV-E7-Polypeptid exprimiert, enthalten ist, oder wobei es zu einem Pulver aus abgetötetem *Lactobacillus casei* hinzugefügt wird.

2. Orale pharmazeutische Zusammensetzung zur Verwendung in einem Verfahren zur Behandlung von HPV-Infektionen nach Anspruch 1, wobei das Kampo-Präparat mit einer revitalisierenden Wirkung Juzentaihoto ist.

3. Orale pharmazeutische Zusammensetzung zur Verwendung in einem Verfahren zur Behandlung von HPV-Infektionen nach einem der Ansprüche 1 bis 2, wobei das HPV-E7-Polypeptid von HPV16 abgeleitet ist.

4. Orale pharmazeutische Zusammensetzung zur Verwendung in einem Verfahren zur Behandlung von HPV-Infektionen nach einem der Ansprüche 1 bis 3, wobei der Patient ein Patient mit einer Vorläuferläsion von Gebärmutterhalskrebs oder ein Patient mit Gebärmutterhalskrebs ist.

5. Eine orale pharmazeutische Zusammensetzung, geeignet zur Behandlung von HPV-Infektionen, die die zelluläre Immunität stimuliert, die spezifisch für ein HPV-E7-Polypeptid in einer Mucosa eines Patienten ist, umfassend:
ein HPV-E7-Polypeptid;
ein Kampo-Präparat mit einer revitalisierenden Wirkung; und
ein Schleimhautadjuvans,
wobei das Kampo-Präparat mit einer revitalisierenden Aktivität Juzentaihoto, Hochuekkito oder beides ist,
wobei das Schleimhautadjuvans ein Polypeptid ist, das von der B-Untereinheit des hitzelabilen Enterotoxins aus *Escherichia coli* oder dem synthetischen Ceramid von α-Galactosylceramid abgeleitet ist, und
wobei das HPV-E7-Polypeptid in einem Pulver aus abgetötetem rekombinantem *Lactobacillus casei,* der das HPV-E7-Polypeptid exprimiert, enthalten ist, oder wobei es zu einem Pulver aus abgetötetem *Lactobacillus casei* hinzugefügt wird.

6. Orale pharmazeutische Zusammensetzung, die zur Behandlung einer HPV-Infektion nach Anspruch 5 geeignet ist, wobei das Kampo-Präparat mit einer revitalisierenden Wirkung Juzentaihoto ist.

7. Orale pharmazeutische Zusammensetzung, die zur Behandlung von HPV-Infektionen nach einem der Ansprüche 5 bis 6 geeignet ist, wobei das HPV-E7-Polypeptid von HPV16 abgeleitet ist.

8. Orale pharmazeutische Zusammensetzung, die zur Behandlung von HPV-Infektionen nach einem der Ansprüche 5 bis 7 geeignet ist, wobei der Patient ein Patient mit einer Vorläuferläsion von Gebärmutterhalskrebs oder ein Patient mit Gebärmutterhalskrebs ist.

## Revendications

1. Composition pharmaceutique par voie orale pour utilisation dans un procédé de traitement d'une infection par le papillomavirus humain (HPV) qui stimule l'immunité cellulaire spécifique d'un polypeptide HPV E7 dans la muqueuse d'une patiente, comprenant :
un polypeptide HPV E7 ;
une préparation Kampo présentant une activité revitalisante ; et
un adjuvant muqueux,
dans laquelle la préparation Kampo présentant une activité revitalisante est le Juzentaihoto, l'Hochuekkito ou les deux,
dans laquelle l'adjuvant muqueux est un polypeptide tiré de la sous-unité B de l'entérotoxine thermolabile provenant d'*Escherichia coli* ou de la céramide synthétique d'α-galactosylcéramide, et
dans laquelle le polypeptide HPV E7 est contenu sous forme de poudre de *Lactobacillus casei* recombinant inactivé, exprimant le polypeptide HPV E7 ou est ajouté à une poudre de *Lactobacillus casei* inactivé.

2. Composition pharmaceutique par voie orale pour utilisation dans un procédé de traitement d'une infection par HPV selon la revendication 1, dans laquelle la préparation Kampo présentant une activité revitalisante est le Juzentaihoto.

3. Composition pharmaceutique par voie orale pour utilisation dans un procédé de traitement d'une infection par HPV selon l'une quelconque des revendications 1 à 2, dans laquelle le polypeptide HPV E7 est tiré de HPV16.

4. Composition pharmaceutique par voie orale pour utilisation dans un procédé de traitement d'une infection par HPV selon l'une quelconque des revendications 1 à 3, dans laquelle la patiente est une patiente présentant une lésion précurseur du cancer du col de l'utérus ou une patiente souffrant d'un cancer du col de l'utérus.

5. Composition pharmaceutique par voie orale adaptée afin de traiter une infection par HPV qui stimule l'immunité cellulaire spécifique à un polypeptide HPV E7 dans la muqueuse d'une patiente, comprenant :
un polypeptide HPV E7 ;
une préparation Kampo présentant une activité revitalisante ; et
un adjuvant muqueux ;
dans laquelle la préparation Kampo présentant une activité revitalisante est le Juzentaihoto, l'Hochuekkito ou les deux,
dans laquelle l'adjuvant muqueux est un polypeptide tiré de la sous-unité B de l'entérotoxine thermolabile provenant d'*Escherichia coli* ou de la céramide synthétique d'α-galactosylcéramide, et
dans laquelle le polypeptide HPV E7 est contenu sous forme de poudre de *Lactobacillus casei* recombinant inactivé exprimant le polypeptide HPV E7 ou est ajouté à une poudre de *Lactobacillus casei* inactivé.

6. Composition pharmaceutique par voie orale adaptée pour le traitement d'une infection par HPV, selon la revendication 5, dans laquelle la préparation Kampo présentant une activité revitalisante est le Juzentaihoto.

7. Composition pharmaceutique par voie orale adaptée pour le traitement d'une infection par HPV selon l'une quelconque des revendications 5 à 6, dans laquelle le polypeptide HPV E7 est tiré de HPV16.

8. Composition pharmaceutique par voie orale adaptée pour le traitement d'une infection par HPV selon l'une quelconque des revendications 5 à 7, dans laquelle la patiente est une patiente présentant une lésion précurseur d'un cancer du col de l'utérus ou une patiente souffrant d'un cancer du col de l'utérus.


### SEQUENCE LISTING

<110> The University of Tokyo
<120> MUCOSAL IMMUNITY POTENTIATOR AND ORAL PHARMACEUTICAL COMPOSITION FOR THE TREATMENT OF HPV INFECTION
<130> NST00413P
<150> JP 2012-128943
   <151> 2012-06-20
<160> 2
<170> PatentIn version 3.5
<210> 1
   <211> 98
   <212> PRT
   <213> Human papillomavirus type 16
<400> 1
   Met His Gly Asp Thr Pro Thr Leu His Glu Tyr Met Leu Asp Leu Gln
   1               5                   10                  15
   Pro Glu Thr Thr Asp Leu Tyr Cys Tyr Glu Gln Leu Asn Asp Ser Ser
               20                  25                  30
   Glu Glu Glu Asp Glu Ile Asp Gly Pro Ala Gly Gln Ala Glu Pro Asp
           35                  40                  45
   Arg Ala His Tyr Asn Ile Val Thr Phe Cys Cys Lys Cys Asp Ser Thr
       50                  55                  60
   Leu Arg Leu Cys Val Gln Ser Thr His Val Asp Ile Arg Thr Leu Glu
   65                  70                  75                  80
   Asp Leu Leu Met Gly Thr Leu Gly Ile Val Cys Pro Ile Cys Ser Gln
                   85                  90                  95
   Lys Pro
<210> 2
   <211> 98
   <212> PRT
   <213> Human papillomavirus type 16
<400> 2
   Met His Gly Asp Thr Pro Thr Leu His Glu Tyr Met Leu Asp Leu Gln
   1               5                   10                  15
   Pro Glu Thr Thr Gly Leu Tyr Gly Tyr Gly Gln Leu Asn Asp Ser Ser
                   20                  25                  30
   Glu Glu Glu Asp Glu Ile Asp Gly Pro Ala Gly Gln Ala Glu Pro Asp
           35                  40                  45
   Arg Ala His Tyr Asn Ile Val Thr Phe Cys Cys Lys Cys Asp Ser Thr
       50                  55                  60
   Leu Arg Leu Cys Val Gln Ser Thr His Val Asp Ile Arg Thr Leu Glu
   65                  70                  75                  80
   Asp Leu Leu Met Gly Thr Leu Gly Ile Val Cys Pro Ile Cys Ser Gln
                   85                  90                  95
   Lys Pro